# EUROPEAN PATENT APPLICATION

(11) **EP 1 961 810 A1**
(43) Date of publication of application: **27.08.2008**
(21) Application number: 08250392.1
(22) Date of filing: 01.02.2008
(51) Int. Cl.: C12N 5/06

(54) **Method for obtaining intestinal stem-precursor cell**

(30) Priority: 02.02.2007 JP 2007024169; 18.09.2007 JP 2007241536
(71) Applicant: Riken, Wako-shi, Saitama 351-0198 (JP)
(72) Inventor: Taniguchi, Hideki, Kobe-shi, Hyogo 650-0047 (JP); Suzuki, Atsushi, Kobe-shi, Hyogo 650-0047 (JP)
(74) Representative: Woods, Geoffrey Corlett

(57) **Abstract**

The present invention relates to a method of acquiring stem/progenitor cells of intestinal epithelial cells and culturing the same, which includes culturing an aggregate of intestinal cells, preferably, culturing the aggregate in a culture broth containing EGF, and a method of inducing differentiation from stem/progenitor cells of intestinal epithelial cells to cells that configure intestinal epithelial tissue, which includes removing EGF from a culture broth of the cells.

## Description

### Technical Field

The present invention relates to a method of acquiring stem/progenitor cells of intestinal epithelial cells and efficiently culturing the same, and to stem/progenitor cells obtained thereby. The present invention also relates to a method of inducing differentiation from stem/progenitor cells of intestinal epithelial cells to cells that configure intestinal epithelial tissue.

### Background Art

In drug absorption studies and other pharmacological investigations, primary culture cells of intestinal epithelial cells are often used. In conventional culture systems, intestinal epithelial cells undergo selection in several days, necessitating constant preparation and use of fresh cells. For this reason, cell lines derived from tumor tissues are used; however, to obtain highly reliable data, it is necessary to use normal cells. Therefore, there is a demand for convenient use of normal intestinal epithelial cells. In this case, in order to maintain cells in culture for a long time, it can be said that stem/progenitor cells that are constantly producing mature intestinal epithelial cells need to be maintained in culture in addition to the mature intestinal epithelial cells.

In the intestine, an organ responsible for food digestion and absorption, stem cells that occupy the fundamental position of a group of intestinal epithelial cells with different functional roles (intestinal stem cells) are present, and intestinal epithelial cells are supplied continuously by division and maintenance of the stem cells. Hence, intestinal stem cells have the capability of vigorous proliferation, but it is extremely difficult to artificially keep the capability in culture; there have been no successful cases of cultivation of intestinal stem cells. If the proliferation and differentiation of intestinal stem cells can be controlled in culture, the intestinal stem cells will be useful as a basic research tool for intestinal stem cell systems, and also useful as a screening tool for drug discovery processes such as drug absorption and metabolism studies. It is also possible to supply intestinal epithelial tissue that is transplantable by tissue engineering.

Meanwhile, embryonic stem cells (ES cells), which represent a pluripotent stem cell line derived from an early embryo, undergo gradual differentiation when cultured by an ordinary method because of the pluripotency thereof. For this reason, ES cells need to be proliferated in the undifferentiated state for efficient proliferation of ES cells. In the case of mouse ES cells, it is known that by adding leukemia inhibitory factor (LIF) to the medium, the undifferentiated state can be maintained (Smith AG, Heath JK, Donaldson DD, Wong GG, Moreau J, Stahl M, Rogers D. Inhibition of pluripotential embryonic stem cell differentiation by purified polypeptides. Nature. 1988 Dec 15;336(6200):688-90.); however, in intestinal stem cells, which are tissue stem cells, no additive factors that can substitute for LIF are known. It is commonly known that ES cells produce a cell aggregate known as the embryoid body during differentiation induction, but no methods for controlling the differentiation of intestinal stem cells have been established.

### Disclosure of the Invention

It is an object of the present invention to acquire stem/progenitor cells of intestinal epithelial cells, and to culture the stem/progenitor cells efficiently. It is another object of the present invention to provide a method of inducing differentiation from stem/progenitor cells of intestinal epithelial cells to cells that configure intestinal epithelial tissue.

The present inventors diligently investigated in view of the above-described problems, and found that by suspension-culturing cells derived from the intestine (intestinal cells) to obtain a cell aggregate, and culturing the aggregate in a culture broth containing epithelial growth factor (EGF), intestinal stem/progenitor cells can be acquired and proliferated selectively. Furthermore, the present inventors succeeded in differentiating the stem/progenitor cells obtained into cells that configure intestinal epithelial tissue under appropriate differentiation induction conditions, and developed the present invention.

Accordingly, the present invention provides:
[1] A method of acquiring stem/progenitor cells of intestinal epithelial cells, comprising culturing an aggregate of intestinal cells.
[2] A method of acquiring stem/progenitor cells of intestinal epithelial cells, comprising culturing an aggregate of intestinal cells in a culture broth containing EGF.
[3] The method according to [1] or [2], wherein the aggregate is obtained by suspension-culturing intestinal cells.
[4] A method of acquiring stem/progenitor cells of intestinal epithelial cells, comprising the following steps:
   (1) a step for collecting intestinal cells from the intestine;
   (2) a step for preparing a cell mass (aggregate) by suspension-culturing intestinal cells;
   (3) a step for culturing an aggregate of intestinal cells in a culture broth containing EGF; and
   (4) a step for recovering cells that are expressing a marker for stem/progenitor cell of intestinal epithelial cells as stem/progenitor cells of intestinal epithelial cells.
[5] The method according to [4] above, wherein the marker for stem/progenitor cells of intestinal epithelial cells is Musashi-1.
[6] A method of acquiring stem/progenitor cells of intestinal epithelial cells, comprising culturing intestinal cells in a culture broth containing serum, insulin, dexamethasone, nicotinamide, L-glutamine, β-mercaptoethanol and EGF.
[7] A method of acquiring stem/progenitor cells of intestinal epithelial cells, comprising culturing intestinal cells at high density in a culture broth containing EGF.
[8] A method of acquiring stem/progenitor cells of intestinal epithelial cells, comprising the following steps:
   (1) a step for collecting intestinal cells from the intestine;
   (2) a step for culturing intestinal cells at high density in a culture broth containing EGF; and
   (3) A step for recovering cells that are expressing a marker for stem/progenitor cell of intestinal epithelial cells as stem/progenitor cells of intestinal epithelial cells.
[9] The method according to [8] above, wherein the marker for stem/progenitor cell of intestinal epithelial cells is Musashi-1.
[10] Stem/progenitor cells of intestinal epithelial cells obtained by the method according to any one of [1] to [9] above.
[11] A method of culturing stem/progenitor cells of intestinal epithelial cells, comprising culturing an aggregate of intestinal cells.
[12] A method of culturing stem/progenitor cells of intestinal epithelial cells, comprising culturing an aggregate of intestinal cells in a culture broth containing EGF.
[13] The method according to [11] or [12] above, wherein the aggregate is obtained by suspension-culturing intestinal cells.
[14] A method of culturing stem/progenitor cells of intestinal epithelial cells, comprising culturing intestinal cells in a culture broth containing serum, insulin, dexamethasone, nicotinamide, L-glutamine, β-mercaptoethanol and EGF.
[15] A method of culturing stem/progenitor cells of intestinal epithelial cells, comprising culturing intestinal cells at high density in a culture broth containing EGF.
[16] A method of inducing differentiation from stem/progenitor cells of intestinal epithelial cells to cells that configure intestinal epithelial tissue, wherein EGF is removed from a culture broth of the stem/progenitor cells.
[17] The method according to [16] above, wherein the cells that configure intestinal epithelial tissue are intestinal secretory cells or mucus-secreting cells.
[18] A medium for culturing stem/progenitor cells of intestinal epithelial cells, containing serum, insulin, dexamethasone, nicotinamide, L-glutamine, β-mercaptoethanol and EGF.

### Brief Description of the Drawings

FIG. 1 is a representation showing brief procedures of intestinal cell culture. "a" is a representation (phase contrast image) showing an aggregate formed on a non-adhesive culture vessel. "b" is a representation (phase contrast image) showing a state of the formed aggregate after being cultured on an ordinary Petri dish without treatment for adhesive cells (but also without treatment for low adhesiveness). "c" is a representation (phase contrast image) showing a state of intestinal cells after being cultured on an adhesive culture vessel without forming an aggregate.
FIG. 2 is a representation (phase contrast image) showing a state of epithelial cell-like cells derived from intestinal cells obtained by a series of procedures, after being proliferated by subculture.
FIG. 3 is a representation (phase contrast images) showing the EGF-dependent proliferation capability of fetal intestine progenitor (FIP) cells. "a" and "b" show states of the cells after being cultured for 2 days in the absence of EGF and in the presence of EGF, respectively. "c" shows cell counting results obtained using a hematology counting chamber after the cells were detached with trypsin-EDTA. The abscissa indicates the concentration of EGF added, and the ordinate indicates the number of cells.
FIG. 4 is a representation (phase contrast images) showing states of cells obtained by culturing FIP cells in the absence of EGF or in the presence of EGF successively after being cultured in the presence of EGF. "a" shows a state of FIP cells after being cultured in the presence of EGF for 2 days. After cultivation in the presence of EGF for 2 days, the cells were further cultured in a culture broth containing EGF for 1 day (b), or in a culture broth not containing EGF for 1 day (c).

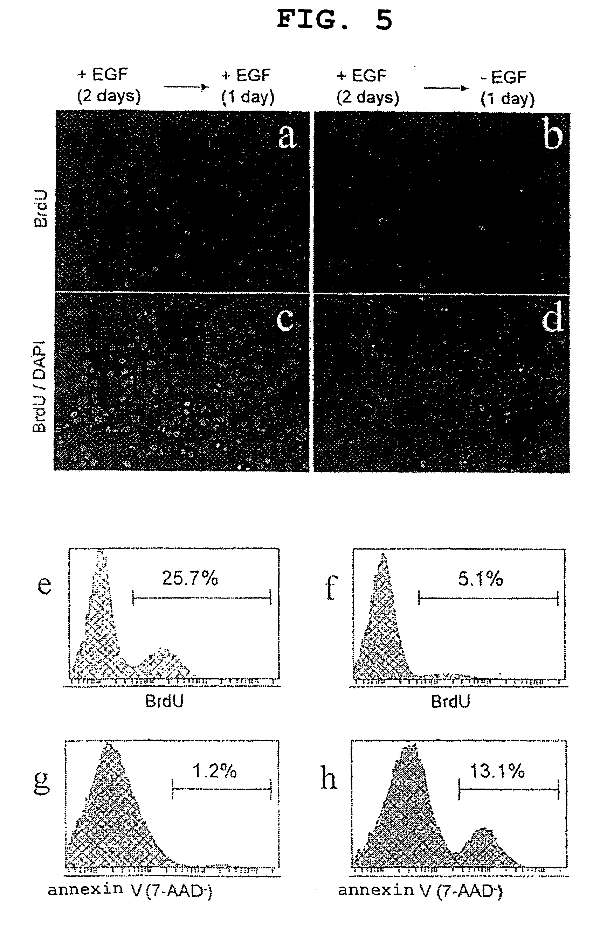

FIG. 5 is a representation showing states of cells obtained by culturing FIP cells in the presence of EGF or in the absence of EGF successively after being cultured in the presence of EGF. "a" and "b" are representations (fluorescence microscopic images) showing results of examinations of BrdU uptake patterns of the respective cells by the BrdU immunological staining method. "c" and "d" are representations (fluorescence microscopic images) showing results of double-staining of the respective cells with BrdU/DAPI. "e" and "f" are graphs showing results of FACS analyses of BrdU uptake in the presence (e) or absence (f) of EGF. "g" and "h" are graphs showing results of FACS analyses of annexin V binding in the presence (g) or absence (h) of EGF.
FIG. 6 is a representation (fluorescence microscopic images) showing results of examinations of the expression of various marker proteins (E-cadherin, Zo-1, villin and Musashi-1) in FIP cells cultured continuously in the presence of EGF.
FIG. 7 is a representation showing results obtained by introducing a construct wherein the β galactosidase gene is driven by a TCF/b-catenin binding site (TOPGAL) into cells, and detecting positive cells by X-gal staining.
FIG. 8 is a representation (phase contrast images) showing states of cells obtained by removing EGF from FIP cells after being cultured in the presence of EGF (a, c), and further culturing the same in the absence of EGF for 7 days (b, d).
FIG. 9 shows PAS-staining images of cells obtained by continuously culturing FIP cells in the absence of EGF (epithelial cell-like cells). "a" shows FIP cells that have become confluent in the presence of EGF; "b" shows cells deprived of EGF and cultured in the absence of EGF for 7 days.
FIG. 10 is a schematic representation showing the relationship between FIP cells and EGF.
FIG. 11 is a representation showing results of examinations of various gene expression profiles in FIP cells, mouse intestinal tissue on day 14.5 of viviparity, neonatal intestinal tissue, and adult intestinal tissue by the RT-PCR method.

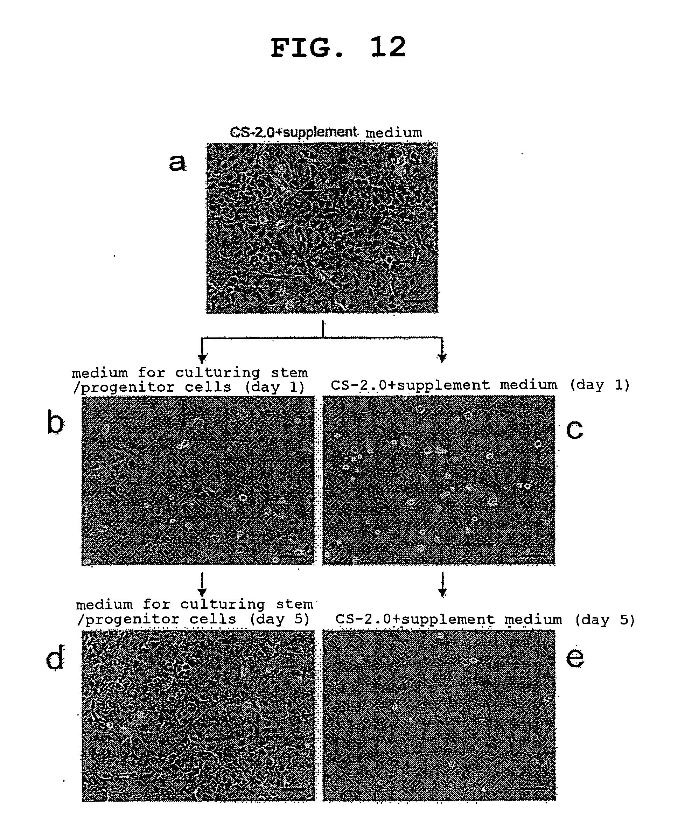

FIG. 12 is a representation showing appearances of cells obtained by subculturing human small intestine epithelial cells (Cat No. CS-ABI-519) using CS-2.0+supplement medium or a medium for culturing intestinal stem/progenitor cells. "a" shows an appearance of the cells sown into CS-2.0+supplement medium after first subculture; "b" and "d" show appearances after second subculture using a medium for culturing intestinal stem/progenitor cells. Shown are appearances on day 1 (b) and day 5 (d) after sowing. "c" and "e" show appearances of the cells after second subculture using CS-2.0+supplement medium. Shown are appearances on day 1 (c) and day 5 (e) after sowing. Phase contrast images.

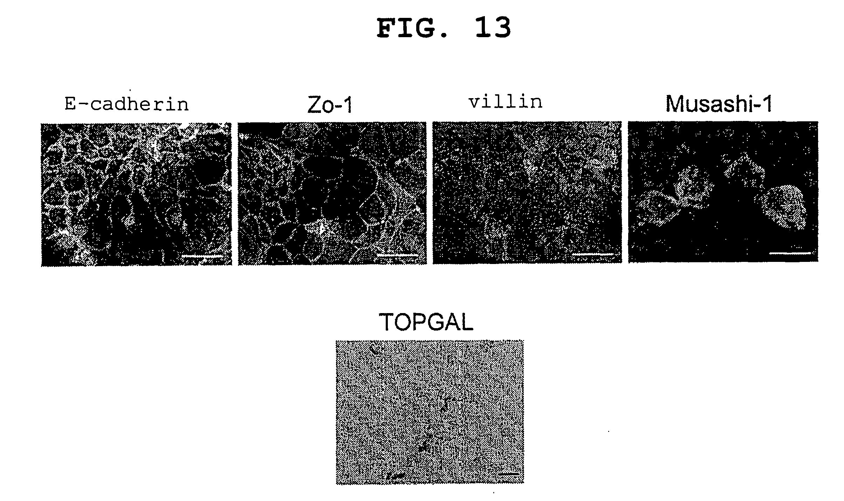

The upper panel of FIG. 13 is a representation (fluorescence microscopic images) showing results of examinations of the expression of various marker proteins (E-cadherin, Zo-1, villin and Musashi-1) in human adult intestine progenitor (hAIP) cells cultured continuously in the presence of EGF. The lower panel is a representation showing results obtained by introducing a construct wherein the β galactosidase gene is driven by a TCF/b-catenin binding site (TOPGAL) into cells, and detecting positive cells by X-gal staining.

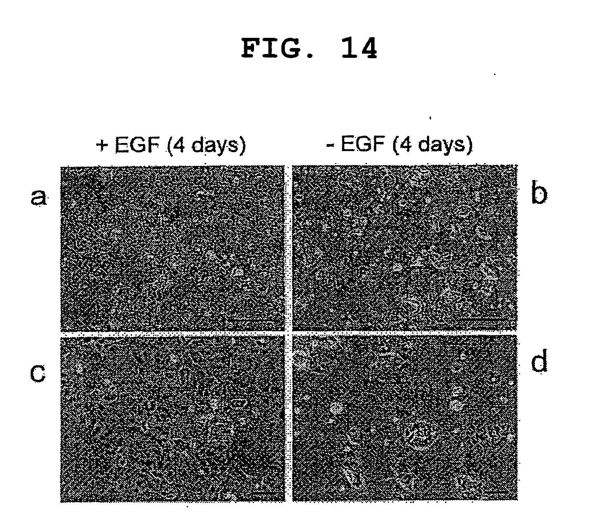

FIG. 14 is a representation (phase contrast image) showing the EGF-dependent proliferation capability of hAIP cells. "a" and "c", and "b" and "d" show states of the cells after being cultured in the presence of EGF, and in the absence of EGF, respectively, for 4 days. "c" and "d" are highly magnified views of "a" and "b", respectively.

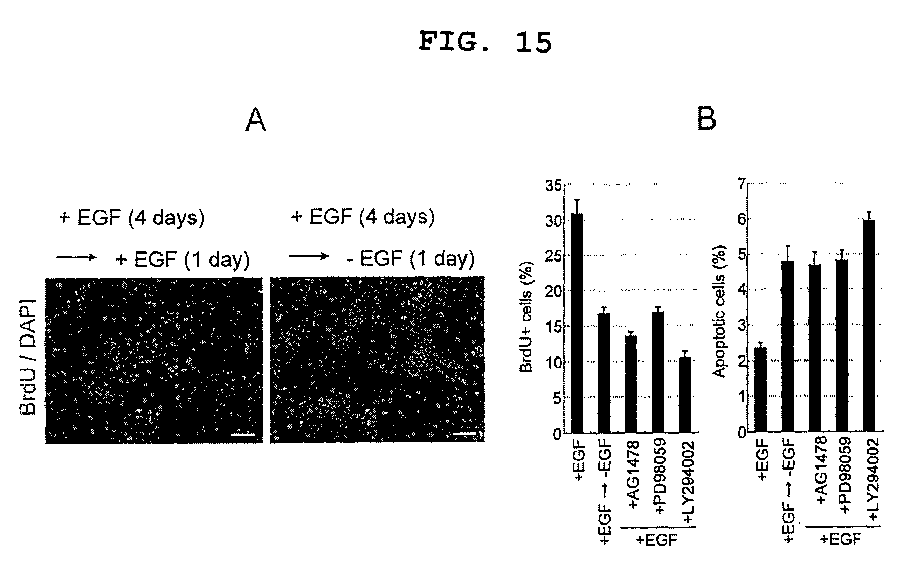

FIG. 15 is a representation showing results of analyses of the effects of EGF on hAIP cell proliferation and apoptosis. FIG. 15A is a representation showing a state of the cells obtained by culturing hAIP cells in the presence of EGF or in the absence of EGF successively after being cultured in the presence of EGF. Shown are results obtained by double-staining the respective cells with BrdU/DAPI (fluorescence microscopic images). FIG. 15B is a graph showing results of FACS analyses of BrdU uptake in the presence or absence of EGF, or with the use of various inhibitors, or results of FACS analyses of cells undergoing apoptosis.

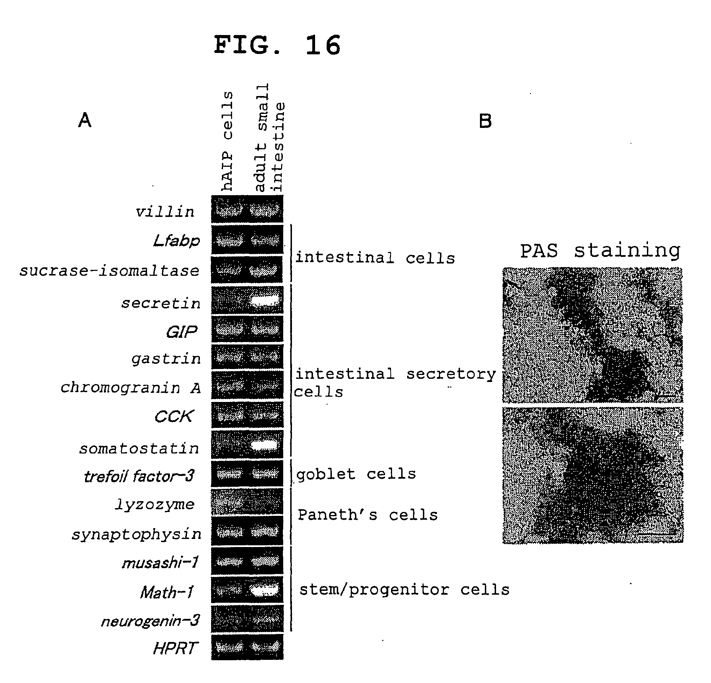

FIG. 16A is a representation showing results of examinations of various gene expression profiles in hAIP cells and human adult intestine tissue by the RT-PCR method.

FIG. 16B is a PAS staining image of hAIP cells.

### Best Mode for Carrying out the Invention

Unless otherwise specified in the text, all technical terms and scientific terms used herein have the same meanings as those commonly understood by those skilled in the art to which the present invention belongs. The same or equivalent optionally chosen methods and materials as those described herein can be used in the embodiments and tests in the present invention; preferable methods and materials are described below. All publications and patents mentioned herein are incorporated herein by reference for the purpose of, for example, describing and disclosing constructs and methodologies described in publications that can be used in relation to the invention described.

Stem/progenitor cells of intestinal epithelial cells to be subjects of the method of acquirement and method of cultivation according to the present invention are derived from intestinal cells of an optionally chosen mammal. Here, "a mammal" specifically includes humans, bovines, horses, dogs, guinea pigs, mice, rats and the like. Although the mammal is preferably a human, animals that are commonly used as laboratory animals, such as mice and rats, are also preferable in consideration of utilization as research tools for drug absorption experiments and the like.

The intestine can roughly be divided into two parts: the small intestine and the large intestine. The small intestine is further divided into the duodenum, the jejunum, and the ileum, from the oral side, and the large intestine is divided into the cecum, the colon (ascending colon, transverse colon, descending colon, sigmoid colon), and the rectum. The adult intestine is configured with lumens surrounded by an epithelial layer folded extensively into lacunae and villi and supportive mesenchymal tissue. Intestinal epithelial cells are constantly renewed, and old ones become detached. Genesis of intestinal epithelial cells occurs because of the presence of a cell regeneration system by stem cells in intestinal epithelial tissue. Stem cells are present in the bottoms of crypts forming depression in villi root of the small intestine. In fetuses, the endodermal cell layer is destined to become intestinal epithelium, transforming into a polarized epithelial monolayer differentiated from a stratified cell layer. The morphology and homeostasis of intestinal epithelium are a result of a highly controlled balance among cell proliferation, migration, differentiation and apoptosis. "Stem/progenitor cells of intestinal epithelial cells" being a subject of the method of acquirement and method of cultivation according to the present invention are cells that are capable of differentiating, and/or destined to differentiate, into intestinal epithelial cells. "Stem cells" are cells having both self-replicating capability and pluripotency, and do not completely coincide with "progenitor cells", which have limited destination for differentiation, but these two share the same identity as cells that differentiate into intestinal epithelial cells; accordingly, in the invention of this application, these two are together referred to as "stem/progenitor cells" without particular distinguishment. "Stem/progenitor cells of intestinal epithelial cells" are also referred to as "intestinal stem/progenitor cells" for the sake of convenience.

As used herein, the term "intestinal cells" refer generically to cells of intestinal origin collected from an adult or a fetus, which cells are intestinal epithelial cells having the capability of division or cells capable of becoming intestinal epithelial cells. Specifically, intestinal cells can be obtained by extirpating and shredding the intestine, preferably the small intestine, and dispersing the cells by physical means such as vibration, or by a chemical treatment using EGTA or EDTA and the like, or by an enzymatic treatment with a protease such as collagenase, trypsin, chymotrypsin, or pepsin (preferably collagenase). These treatments may be performed singly, or in combination of a plurality of treatments. If the fetal intestine is used, it is preferable that intestinal cells be collected at a time when undulation begins to occur in intestinal epithelial tissue from the cylindrical intestinal structure. In mice, for example, intestinal cells are collected from a fetus on day 12 to 17 of viviparity, preferably on day 14.5. As used in the present invention, "intestinal cells" may be collected from the intestine as described above, and may also be commercially available cells derived from the intestine containing intestinal epithelial cells having the capability of division or cells capable of becoming intestinal epithelial cells. Such commercially available cells derived from the intestine include human small intestine epithelial cells separated by ACBRI (Applied Cell Biology Research Institute) (ACBRI 519), and available in Japan from Dainippon Sumitomo Pharma Co., Ltd. (human intestinal epithelial cells, Cat No. CS-ABI-519).

A first feature of the present invention resides in allowing intestinal cells to form an aggregate, and culturing the aggregate. By allowing the cells to form an aggregate, the cells can be brought into an environment more similar to that in vivo. Here, "an aggregate" refers to a cell mass having a three-dimensional structure (spherical or grape-cluster-like) formed by a large number of cells gathering together. For example, as described below, if cells are sown at a density of 200000 to 400000 cells/well to a low adhesive 96-well round-based dish, about 10 to 20 aggregates having a diameter of about 200 to 500 µm are formed. A sheet-like structure of cells is observed in the outermost part of each aggregate. As long as an environment more similar to that in vivo can be provided, the number and size of the cells that configure each aggregate are variable depending on the state of the cells and culture conditions.

Aggregates of intestinal cells can specifically be prepared as described below. First, intestinal cells are suspension-cultured in an appropriate medium on a low adhesive culture vessel. For example, if a 96-well round-based dish is used as the low adhesive culture vessel, 200000 to 400000 cells per well are sown, and the aggregates formed are cultured in an appropriate medium at 37°C for 2 to 10 days, preferably 7 days, while transferred to a low adhesive 6-cm dish and the like. The low adhesive culture vessel is exemplified by a culture vessel in common use in the art subjected to a treatment for low adhesiveness. Culture vessels include culture dishes, culture flasks, rotary culture devices (spinner flasks and the like) and the like. Useful treatments for low adhesiveness include a treatment to suppress the adhesion of proteins and cells by covalently binding hydrogel (coating). Commercially available ones can also be used.

Although adult intestinal cells are unlikely to form aggregates, a similar effect (the effect of creating an environment similar to that in vivo) can be obtained by culturing the intestinal cells at high density. For example, if cultivation is performed using a Petri dish having a diameter of 6 cm (amount of medium: 3.5 mL) (an ordinary Petri dish without treatment for adhesive cells (but also without treatment for low adhesiveness); hereinafter, also simply referred to as an ordinary Petri dish), high-density culture can be accomplished by sowing 1 to 5X10⁶ cells, and culturing the same in an appropriate medium at 37°C for 1 to 5 days, preferably 3 days.

The medium that can be used in the above-described culture of aggregates and high-density culture is not particularly limited, as long as intestinal cells can survive, and the state wherein differentiation and apoptosis induction are suppressed can be maintained; useful media include media based on cell culture media in common use in the art (Dulbecco's modified Eagle medium, MEM, RPMI1640 medium, Ham's F-12 medium and the like), and supplemented with various ingredients as required. Particularly, it is preferable that the base medium be supplemented with ingredients such as serum, antibiotics, and growth factor.

Suitable ingredients to be contained in the medium include 5 to 20%, preferably 10% serum, 0.5 to 2 µg/mL, preferably 1 µg/mL insulin, 1X10⁻⁸ to 1X10⁻⁶ mol/L, preferably 1X10⁻⁷ mol/L dexamethasone, 5 to 15 mmol/L, preferably 10 mmol/L nicotinamide, 1 to 5 mmol/L, preferably 2 mmol/L L-glutamine, 10 to 100 µmol/L, preferably 50 µmol/L β-mercaptoethanol, penicillin/streptomycin and the like. All are commercially available. The serum is exemplified by equine or bovine-derived serum and the like, and is preferably fetal serum. Although the insulin used may be any of insulin produced from the bovine or swine pancreas and insulin produced by gene recombination technology, it is preferable, from the viewpoint of stable quality and supply, that recombinant insulin be used.

A second feature of the present invention resides in culturing intestinal cells in the presence of EGF (epithelial growth factor) in order to proliferate, acquire or culture intestinal stem/progenitor cells selectively.

EGF was isolated and identified as a substance that promotes early eyelid fission in fetal mice from the mouse submandibular gland by Cohen et al., and then human EGF was discovered in urine (S. Cohen and G. Carpenter et al., Proc. Natl. Acad. Sci. USA., 72, 1317, 975; H. Gregory, Nature, 257, 325, 1975). The gene structure thereof has been elucidated in detail (G.I. Bell et al., Nucleic Acids Res., 14, 8427, 1986). EGF is a 6045-Da protein consisting of 53 amino acid residues and three intramolecular disulfide bonds, binding as a ligand to EGFR (epithelial growth factor receptor) on the cell surface to play an important role in the regulation of cell growth and proliferation. The EGF used in the present invention is not particularly limited with respect to the derivation thereof, as long as it enables the selective proliferation, acquirement or cultivation of intestinal stem/progenitor cells; the EGF may be of natural derivation, or may be produced synthetically or semi-synthetically on the basis of commonly known gene sequences, amino acid sequences and other information. The EGF may also be a commercially available one. Although it is preferable that mouse EGF and human EGF be used in cases where the subject is mouse stem/progenitor cells or human stem/progenitor cells, respectively, it is not always necessary to have the same animal species. As long as intestinal stem/progenitor cells can selectively be proliferated, acquired or cultured, the EGF may be a modified one. Such modifications include mutations, insertions, deletions and substitutions of one or a plurality of nucleotides or amino acids. Modified EGF can be prepared by a means in common use in the art. For example, EGF DNA may be artificially modified by a method of site-directed mutagenesis such as deletion mutagenesis using exonuclease or cassette mutagenesis, and a desired protein can be prepared using the modified EGF DNA.

EGF is preferably persistently contained in the medium through the process of culturing intestinal cells to acquire intestinal stem/progenitor cells; in particular, during the period of forming aggregates, and thereafter culturing and proliferating the same for a long time, EGF is essentially contained in the medium. The concentration of EGF in the medium is about 20 to 40 ng/mL, preferably about 20 ng/mL.

By performing cultivation in a culture broth containing EGF, intestinal stem/progenitor cells can also be obtained from commercially available intestinal cells, for example, the above-described human small intestine epithelial cells (Cat No. CS-ABI-519). If commercially available human small intestine epithelial cells are used, intestinal stem/progenitor cells can be acquired, without the need for aggregate formation and high-density culture, by subculturing the cells using a culture broth containing EGF, particularly a culture broth containing 5 to 20%, preferably 10% serum, 0.5 to 2 µg/mL, preferably 1 µg/mL insulin, 1X10⁻⁸ to 1X10⁻⁶ mol/L, preferably 1X10⁻⁷ mol/L dexamethasone, 5 to 15 mmol/L, preferably 10 mmol/L nicotinamide, 1 to 5 mmol/L, preferably 2 mmol/L L-glutamine, and 10 to 100 µmol/L, preferably 50 µmol/L β-mercaptoethanol, in addition to 20 to 40 ng/mL, preferably 20 ng/mL EGF.

Dulbecco's modified medium - Ham's F12 mixed medium (1:1) supplemented with 10% fetal bovine serum, insulin (1 µg/mL), dexamethasone (1X10⁻⁷ mol/L), nicotinamide (10 mmol/L), L-glutamine (2 mmol/L), β-mercaptoethanol (50 µmol/L), penicillin (100 U/mL)+streptomycin (100 µg/mL) and EGF (20 ng/mL) is a suitable medium (culture broth) for culturing intestinal cells.

The thus-obtained cell aggregates are re-sown to an ordinary Petri dish, and cultured in an EGF-containing medium (culture broth) at 37°C for about 2 to 6 months, preferably about 3 months. Since cells derived from the adult intestine are unlikely to form aggregates, high-density culture is performed to create a state equivalent to cell aggregation. Cells derived from the adult intestine are subjected to high-density culture in a culture broth containing EGF at 37°C for about 6 to 12 months, preferably about 8 months.

If cultivation of aggregates or high-density culture is continued for a long time, a larger number of epithelial cell-like cells emerge compared with fibroblast-like cells. If fibroblast-like cells remain, the fibroblast-like cells only can be killed by treatment with about 1 to 10 µg/mL, preferably 4 µg/mL monoiodoacetic acid, for about 3 to 5 hours. If proliferation of epithelial cell-like cells is observed, the cells are then subcultured in a culture vessel for cell culture, and used for analysis or stored under freezing until use.

A judgment to determine whether the cells obtained by continuing cultivation of aggregates or high-density culture for a long time, or the cells obtained by subculturing commercially available human small intestine epithelial cells such as Cat No. CS-ABI-519 using a culture broth containing EGF, are intestinal epithelial cells or intestinal stem/progenitor cells, can be made by determining the presence or absence of the expression of a marker protein in the cells. As used herein, the term "marker" means each group of a series of proteins exhibiting a mode of expression characteristic of intestinal epithelial cells or intestinal stem/progenitor cells, or genes that encodes the same, unless otherwise specified. Although the marker protein sometimes has the amino acid sequence thereof differing depending on the mammalian species and the like, such proteins can also be used as marker proteins in the present invention, as long as they share the same mode of expression in the subject cells. Marker proteins for epithelial cells include E-cadherin and Zo-1 (Laprise P, et al., J Biol Chem. 2004 Mar 12; 279(11):10157-66. Epub 2003 Dec 29.; Escaffit F, et al., Exp Cell Res. 2005 Jan 15;302(2):206-20); marker proteins for intestinal epithelial cells include villin (Maunoury, R. et al., EMBO J. 7, 3321-3329 (1988)). Marker proteins for intestinal stem/progenitor cells include Musashi-1 (Potten CS, et al., Differentiation. 2003 Jan;71(1):28-41.), Math-1 (Yang Q, et al., Science 294:2155-2158), and Neurogenin 3 (Ngn3, Jenny M, et al., EMBO J. 2002 DEC 2;21(23):6338-47).

E-cadherin, a calcium-dependent intercellular adhesion molecule expressed on epithelial cells, exhibits an important function in the formation and maintenance of epithelial tissue by homophilic adhesion, in which molecules of the same kind bind together. Zo-1 is known as a scaffold protein for the tight junction (TJ), an intercellular adhesion apparatus essential for epithelial cells and endothelial cells, and is thought to be involved in the formation of the adhesion apparatus and the regulation of cell proliferation mediated thereby. Villin, a component of the microvilli of small intestine epithelial cells, functions to bundle actin along with fibrin. Villin is found only in microvilli. Musashi-1 is an RNA-binding protein expressed by nervous system progenitor cells, and has also been reported to be expressed in intestinal stem/progenitor cells. Math-1, a bHLH type transcription factor, is known to be necessary for differentiation into the secretory cell system in the mouse intestine, and is expressed in intestinal stem/progenitor cells. Ngn3, also a bHLH type transcription factor, is important to the genesis of pancreatic secretory cells and gastrointestinal tissue, and is expressed in intestinal stem/progenitor cells.

In the present invention, the confirmation of being intestinal epithelial or intestinal stem/progenitor cells can be performed by including a step of analyzing the expression of such marker protein and/or a gene encoding the same, using a substance having specific affinity for each marker protein or a gene encoding the same. In the present specification, the term "using" is not particularly limited as regards its method. Specifically, when a substance having specific affinity for, for example, a marker protein, is used, a method utilizing an antigen-antibody reaction with the antibody of said marker protein can be mentioned, and when a substance having specific affinity for a gene encoding a marker protein is used, a method utilizing a hybridization reaction can be mentioned.

As a substance having specific affinity for a marker protein, for example, an antibody having specific affinity for the protein, and a fragment thereof can be mentioned, where the specific affinity refers to an ability to specifically recognize the protein by an antigen-antibody reaction and to bind therewith. The antibody and a fragment thereof are not particularly limited as long as they can bind specifically with the protein, and may be a polyclonal antibody, a monoclonal antibody or a functional fragment thereof. These antibodies and their functional fragments can be produced according to a method generally employed in this field. For example, when a polyclonal antibody is used, a method wherein the protein is injected subcutaneously to the back of or intraperitoneally, intravenously and the like to an animal such as mouse and rabbit for immunization, and after awaiting an increase in the antibody titer, antiserum is harvested, can be mentioned. When a monoclonal antibody is used, a method wherein a hybridoma is prepared according to a conventional method, and secretion therefrom is harvested can be mentioned. As a method of producing an antibody fragment, a method wherein a fragment of a cloned antibody gene is expressed in a microorganism and the like is frequently used. The purity of the antibody, antibody fragment and the like is not particularly limited as long as it retains specific affinity for the protein. These antibodies and fragments thereof may be labeled with a fluorescent substance, an enzyme, a radioisotope and the like.

Furthermore, commercially available ones may be used.

A substance having specific affinity for a gene encoding a marker protein is exemplified by oligo- or polynucleotide probe having specific affinity for the gene (hereinafter to be conveniently referred to simply as a probe), and an oligo- or polynucleotide primer pair having specific affinity for the gene (hereinafter to be conveniently referred to simply as a primer pair) can be mentioned. The "specific affinity" thereof refers to a property to hybridize only to the objective gene. Accordingly, it may be completely complementary to all or a part of the gene, or may include one to several mismatches as long as the above-mentioned property is satisfied. The probe and the primer pair are free of any particular limitation as long as they have specific affinity for the gene. For example, an oligo- or polynucleotide containing all or a part of the base sequence of the gene, a complementary sequence thereof, and the like can be mentioned, which are appropriately selected according to the form of the gene to be detected. The oligo- or polynucleotide is not particularly limited as to its origin as long as it has specific affinity for the gene. It may be a synthesized one or one obtained by cleaving out a necessary portion from the gene and purifying by a method generally employed. These oligo- and polynucleotides may be labeled with a fluorescence substance, an enzyme, a radioisotope and the like.

Because β-catenin transcription activity is seen in intestinal stem/progenitor cells (He XC, et al., Nat Genet. 2004 OCT;36(10):1117-21), identity as stem/progenitor cells can also be confirmed with this activity as an index. For example, it is possible to utilize a method comprising introducing a construct wherein the β galactosidase gene is driven by a TCF/b-catenin binding site (TOPGAL) into cells, and detecting positive cells by X-gal staining.

In the present invention, by culturing an aggregate of intestinal cells (or cells after high-density culture) in a culture broth containing EGF, intestinal stem/progenitor cells can be proliferated selectively, that is, the stem/progenitor cells can efficiently be acquired. Furthermore, the self-replicating capability and pluripotency of the stem/progenitor cells are stably maintained for a long time. The maintenance of the self-replicating capability by the intestinal stem/progenitor cells obtained by the method of the present invention can easily be confirmed by observing the proliferation of intestinal stem/progenitor cells in the culture vessel using various commonly known techniques enabling a measurement of cell proliferation, such as cell counting and examination under a light microscope. The maintenance of the pluripotency by the intestinal stem/progenitor cells can be confirmed by determining whether or not a plurality of kinds of cells that configure intestinal epithelial tissue are obtained when the differentiation of the stem/progenitor cells are induced. Cells that configure intestinal epithelial tissue include intestinal secretory cells (gastrin-secreting cells, serotonin-secreting cells, chromogranin A-secreting cells, secretin-secreting cells, somatostatin-secreting cells, GIP (gastric inhibitory polypeptide)-secreting cells, CCK (cholecystokinin)-secreting cells and the like), mucus-secreting cells (goblet cells, Paneth's cells and the like), and intestinal cells responsible for nutrient absorption. By detecting an ingredient expressed specifically in each type of cell using a substance having specific affinity therefor, or by directly analyzing the secreted ingredient, it can be determined whether the cells are desired cells. As the "substance having specific affinity", an antibody having specific affinity for each secreted ingredient or a fragment thereof and an oligonucleotide or polynucleotide probe having specific affinity for the gene that encodes each secreted ingredient can be mentioned. Details of "antibody or a fragment thereof" and "oligonucleotide or polynucleotide probe" are as described above.

Regarding goblet cells, a determination can also be made by staining methods such as PAS staining and Alcian Blue staining.

Induction of the differentiation of intestinal stem/progenitor cells into cells that configure intestinal epithelial tissue can easily be performed by removing EGF from the culture broth. Specifically, this induction can be achieved by medium exchange to replace the culture broth containing EGF with a culture broth not containing EGF. Induction of the differentiation of the cells can be confirmed in about 1 week after medium exchange; the time taken to induce differentiation is variable depending on the state of the cells, the type of differentiating cells, and the like.

### Examples

The present invention is hereinafter described in more detail by means of the following examples, which, however, never limit the scope of the invention. Reagents, apparatuses and materials used in the present invention are commercially available unless otherwise specified.

### Example 1: Acquirement of intestinal stem/progenitor cells derived from fetal intestine

The intestine was extirpated from a fetal mouse on day 14.5 of viviparity, and shredded with a surgical knife to obtain intestine tissue pieces. The intestine tissue pieces obtained were placed in Hank's solution containing 1 mM EGTA, and shaken in a 37°C water bath for 10 minutes, after which the solution was centrifuged and the tissue pieces were once washed with PBS. After centrifugation, a 1 mg/mL collagenase solution (dissolved in Hank's solution containing 5 mM CaCl₂) was added to the intestine tissue pieces, and the solution was shaken in a 37°C water bath for 15 to 20 minutes. After gentle pipetting, the solution was passed through meshes to remove undigested tissue and obtain a cell dispersion (fetal mouse intestinal cells).

The prepared fetal mouse intestinal cells were sown to a low adhesive 96-well round-based dish (Low Cell Binding Plates, cat no: 145399; Nunc) at a density of 2X10⁵ to 4X10⁵ cells/well, and cultured in a culture broth containing Dulbecco's modified Eagle medium and F-12 medium in the 1:1 ratio [containing 10% fetal bovine serum, 1 µg/mL insulin, 1X10⁻⁷ mol/L dexamethasone, 10 mmol/L nicotinamide, 2 mmol/L L-glutamine, 50 µmmol/L β-mercaptoethanol, penicillin/streptomycin (100 U/mL (penicillin)+100 µg/mL (streptomycin)), and 20 ng/mL EGF (Sigma, E9644)] at 37°C in the presence of 5% CO₂ in an incubator. Hereinafter, this medium containing EGF is also referred to as EGF(+) medium or the medium for intestinal stem/progenitor cell culture.

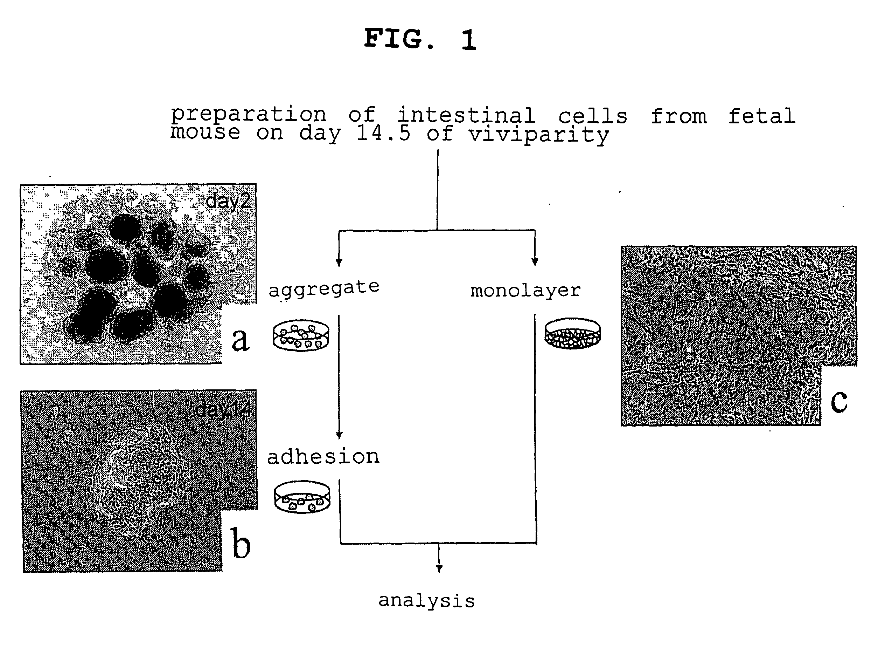

Two days later, the fetal mouse intestinal cells sown to the 96-well round-based dish gathered to form cell masses (FIG. 1a). Thereafter, the cell masses formed were further cultured for about 5 days while transferred to a low adhesive 6-cm dish and the like, after which these cell masses were transferred to a Petri dish (BD, 35-1007), and continued to be cultured in the above-described EGF(+) medium at 37°C in the presence of 5% CO₂ in an incubator (FIG. 1b). The Petri dish used here was an ordinary Petri dish without treatment for adhesive cells (but also without treatment for low adhesiveness). Meanwhile, when the cell dispersion was immediately sown to an adhesive Petri dish and cultured in the above-described EGF(+) medium, without using a low adhesive dish, only fibroblast-like cells remained in the dish (FIG. 1c).

The aggregates were transferred to an ordinary Petri dish and continued to be cultured for about 3 months; a larger number of epithelial cell-like cells emerged compared with fibroblast-like cells. If fibroblast-like cells remain, the fibroblast-like cells can be killed by treatment with monoiodoacetic acid (4 µg/mL) for about 3 to 5 hours.

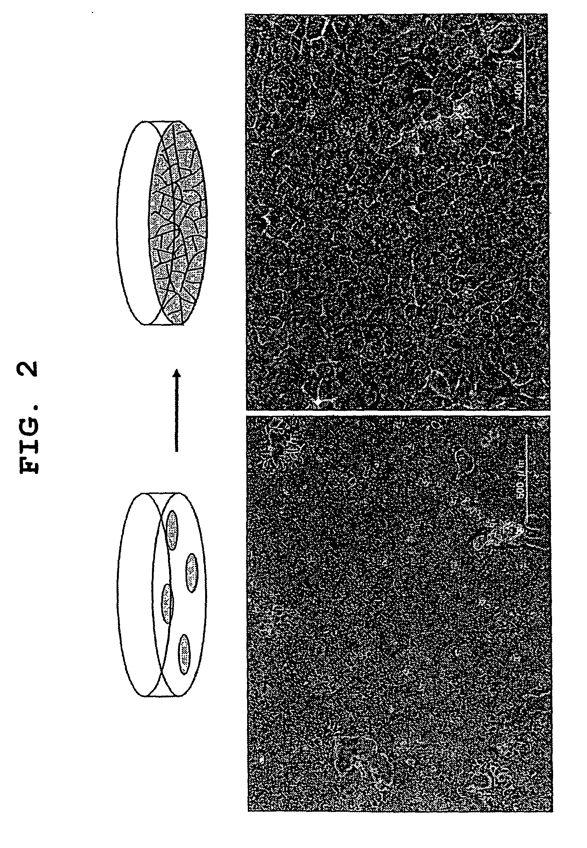

The epithelial cell-like cells obtained were subcultured in dishes for cell culture (FIG. 2), and used for analysis (described below) or stored under freezing.

As is evident from an Example below, these epithelial cell-like cells contain stem/progenitor cells having self-replicating capability and pluripotency, and are therefore hereinafter also referred to as FIP (fetal intestine progenitor) cells for the sake of convenience.

### Example 2: Acquirement of intestinal stem/progenitor cells derived from adult intestine

The inside of the intestine extirpated from an adult mouse was washed with PBS using a 23G needle, after which the intestine was opened with scissors to obtain a sheet, which was cut with scissors into pieces about 5 mm square (intestine tissue pieces). The intestine tissue pieces obtained were treated with EGTA and collagenase, and by pipetting, in the same manner as Example 1, to yield a cell dispersion (adult mouse intestinal cells).

Since adult-derived intestinal cells are unlikely to form cell masses, the cells were cultured while allowing them to aggregate in a monolayer. Specifically, 1X10⁶ to 5X10⁶ cells were sown to a Petri dish having a diameter of 6 cm (an ordinary Petri dish without treatment for adhesive cells (but also without treatment for low adhesiveness)), and high-density culture was performed. Using a culture broth with the same composition as Example 1, the cells were cultured at 37°C in the presence of 5% CO₂ in an incubator.

When high-density culture was continued for about 8 months, a larger number of epithelial cell-like cells emerged compared with fibroblast-like cells. If fibroblast-like cells remain, the fibroblast-like cells can be killed by treatment with monoiodoacetic acid (4 µg/mL) for about 3 to 5 hours.

The epithelial cell-like cells obtained were subcultured in dishes for cell culture, and used for analysis or stored under freezing.

### Example 3: Proliferation capability

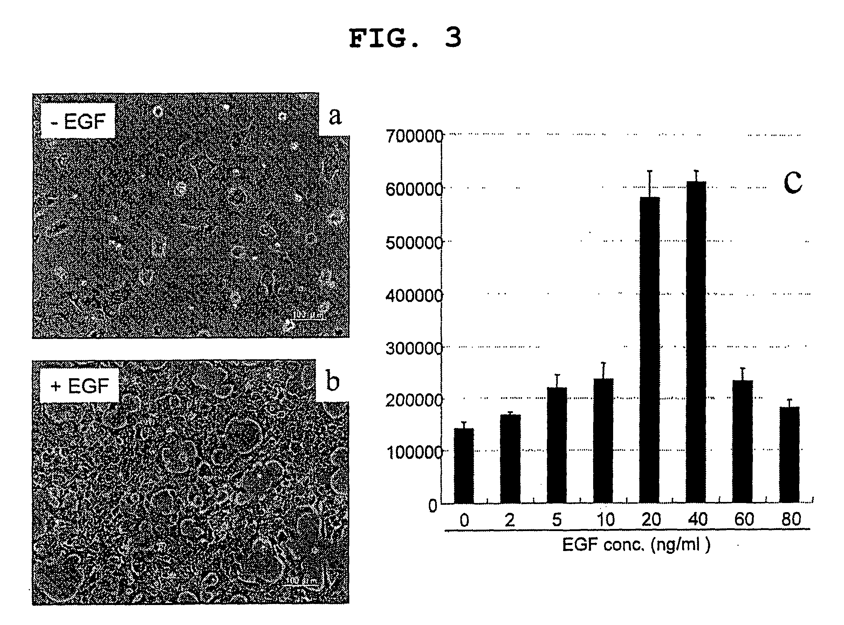

The proliferation capability of FIP cells was examined. The FIP cells were cultured in a culture broth containing EGF at various concentrations (0 ng/mL to 80 ng/mL). The culture broth had the same composition as the culture broth used in Example 1, except that the concentration of EGF differed. Shown are the cells after being cultured in an EGF-free culture broth for 2 days (FIG. 3a) and the cells after being cultured in a culture broth containing EGF at a concentration of 20 ng/mL for 2 days (FIG. 3b). The cells were sown to a 12-well culture plate, and cultured in a culture broth containing EGF at various concentrations for 1 day, after which the cells were detached using trypsin-EDTA, and the number thereof was counted using a hematology counting chamber (FIG. 3c).

It was shown that the proliferation of epithelial cell-like cells derived from intestinal cells is dependent on EGF.

### Example 4: Proliferation and apoptosis

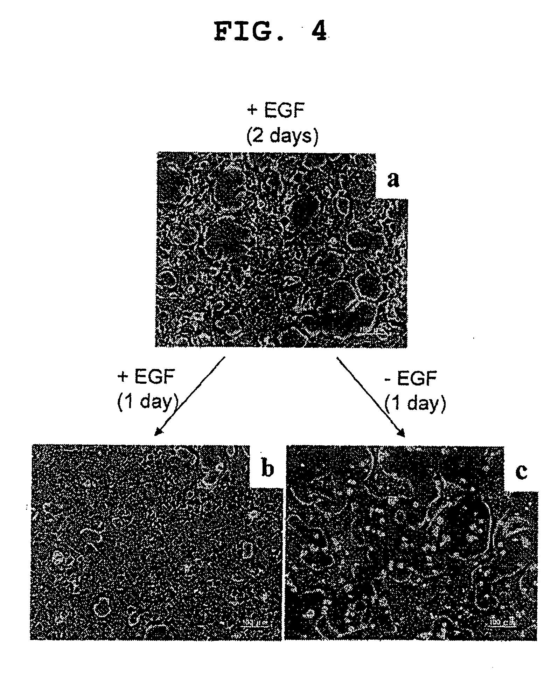

FIP cells were cultured in EGF(+) medium (EGF concentration 20 ng/mL) on a dish for cell culture for 2 days (FIG. 4a). Two sets in the same state were provided. After cultivation in the presence of EGF for 2 days, for one set, the medium was exchanged with the same EGF(+) medium and the cells were further cultured for 1 day (FIG. 4b); for the other set, the medium was exchanged with an EGF-free culture broth and the cells were further cultured for 1 day (FIG. 4c). The cells continued to proliferate in the presence of EGF; however, in the absence of EGF, the proliferation of the cells was suppressed and dead cells were observed. Changes in the proliferation capability of the cells in the series of processes were confirmed by the BrdU immunological staining method. The cells were double-stained with the nucleus staining agent DAPI (FIGS. 5a to f).

After cultivation in the presence of EGF for 2 days, for one set, the medium was exchanged with an EGF-free culture broth and the cells were further cultured for 1 day; for the other set, the medium was exchanged with the same EGF(+) medium and the cells were further cultured for 1 day. Thereafter, BrdU (10 µM) (Sigma, B9285) was added to the medium; 1.5 hours later, the ratio of proliferated cells incorporating BrdU was analyzed by flowcytometry and immunological staining. Flowcytometry analysis was performed using FACS Aria (BD) after the cells were labeled as directed in the manual for the BrdU Flow Kit (BD, 552598). In this case, gate settings were made with a negative control as an index. Immunological staining of BrdU was performed using the reagent for the BrdU Flow Kit, an anti-BrdU antibody (X50) (BD, 347580), a Cy3-conjugated donkey anti-mouse IgG antibody (X200) (Jackson Immunoresearch Laboratories, cat no. 715-165-150), and DAPI (1 µg/mL) (Sigma, D9542), and the cells were examined by fluorescence microscopy. It was found that the cells cultured in the absence of EGF exhibited remarkably decreased BrdU uptake and decreased proliferation capability (FIGS. 5a to f).

When EGF was removed from the culture broth, dead cells were observed; apoptosis induction in the absence of EGF was examined (FIGS. 5g to h).

The cells were cultured in the same manner as BrdU analysis. After the cells were recovered from the dish, the cells were once washed with cold PBS, and a cell suspension was prepared with annexin V binding buffer (1X) (BD, 556454). 7-AAD (5 µL; BD, 559925) and APC-conjugated annexin V (5 µL; BD, 550474) were added thereto, and the reaction was carried out at room temperature for 15 minutes (shaded). The stained cells were analyzed using FACS Aria (BD). Gate settings were made with a negative control as an index. Annexin V is a phospholipid-binding protein. Cells that have undergone apoptosis have phosphatidylserine exposed to the cell membrane surface thereof, and annexin V binds thereto. Therefore, annexin V possesses strong affinity for apoptosis cells. It was shown that in the absence of EGF, annexin V-binding cells increased remarkably, and apoptosis induction occurred (FIGS. 5g to h).

FIP cells exhibited vigorous division and proliferation in the presence of EGF; however, under EGF-free culture conditions, the proliferation activity decreased remarkably, and cells undergoing apoptosis increased.

### Example 5: Expression of marker proteins

The expression of various marker proteins in FIP cells was examined. The expression of each marker protein was examined by an immunological staining method using the antibody against each protein. In order to visualize the cells, the cells were double-stained with the nucleus staining agent DAPI.

The cells were washed with PBS and fixed with 4% paraformaldehyde (PFA) (in PBS) at room temperature for 5 minutes. Subsequently, the cells were treated with 25% acetone (in methanol) at room temperature for 1 minute. After the fixation, the cells were washed with PBS containing 0.05% polyoxyethylene (20) sorbitan monolaurate (Tween 20) (Wako) (PBS-Tween), and subsequently treated with 0.2% Triton X-100 (Sigma) at room temperature for 1 hour. Non-specific binding was blocked by treatment with 10% donkey serum (Sigma, D9663) at room temperature for 1 hour. The fixed cells were incubated with a 1st antibody in a humid chamber at 4°C for 16 hours. The cells were washed with PBS-Tween 20; after blocking, the cells were further incubated with a 2nd antibody at 4°C for 4 hours. The cells were incubated with DAPI at room temperature for 5 minutes and washed, after which the cells were examined by fluorescence microscopy.

The antibodies used are as follows:
(1st antibodies)
   Anti-E-cadherin antibody: BD, 610181 (2nd antibody, for mice); X100
   Anti-Zo-1 antibody: Zymed, 61-7300 (2nd antibody, for rabbits); X100
   Anti-villin antibody: Santa Cruz, SC-7672 (2nd antibody, for goat); X10
   Anti-Musashi-1 antibody: Chemicon, AB5977 (2nd antibody, for rabbits); X100
(2nd antibodies)
   For mice: Alexa 488-conjugated donkey anti-mouse IgG (Molecular Probes, A21202); X200
   For rabbits: Alexa 488-conjugated donkey anti-rabbit IgG (Molecular Probes, A21206); X200
   For goat: Alexa 488-conjugated donkey anti-goat IgG (Molecular Probes, A11055); X200

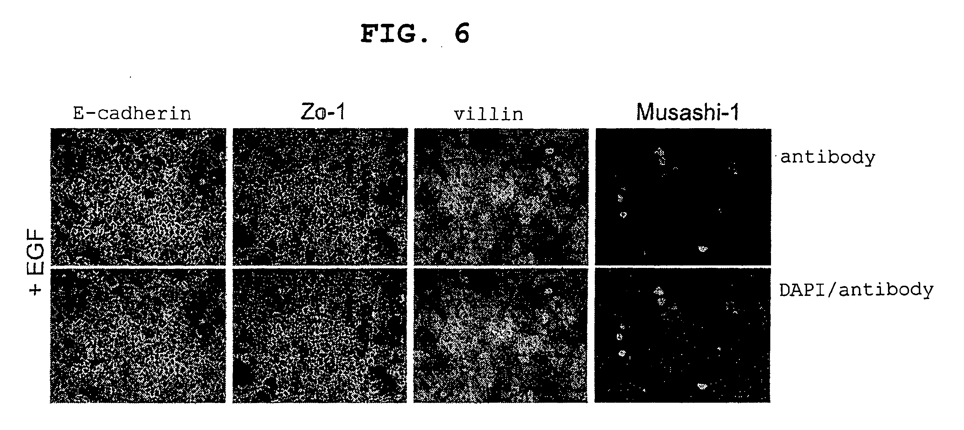

All FIP cells expressed E-cadherin and Zo-1, which are markers of epithelial cells, and villin, a marker of intestinal epithelial cells. Some cells expressed Musashi-1, which is thought to be an intestinal stem/progenitor cell marker (FIG. 6).

### Example 6: β catenin transcription activation

A construct (TOPGAL) wherein the β galactosidase gene is driven by a TCF/b-catenin binding site was introduced into cells using lipofectamine 2000 (Invitrogen, 11668), and positive cells were detected by X-gal staining. Specifically, the following procedures were taken:
(1) Wash cells with PBS (X2).
(2) Add fixative (2% PFA/0.2% glutaraldehyde (GA)/0.05% NP-40/PBS) (1.5 to 2 mL per well of 6-well plate).
(3) Allow the cells to stand at room temperature for 1 minute.
(4) Wash with PBS (X2).
(5) Add X-gal solution (1.5 to 2 mL per well of 6-well plate).
(6) Allow the cells to stand in 37°C incubator (at least 3 hours to overnight).
(7) Wash with PBS (X2) to stop the reaction.
(8) Examine (take photographs) and store in PBS at 4°C.

### (Preparation of fixative)

The fixative was prepared by mixing each ingredient in the following ratio.

| | |
|---|---|
| 20% PFA/PBS | 1 mL (stored at -20°C) |
| 25% GA | 80 µL (stored at -20°C) |
| 5% NP-40 | 80 µL (stored at room temperature) |
| PBS | 9 mL (stored at room temperature) |

### (Preparation of X-gal solution)

The X-gal solution was prepared by mixing each ingredient in the following ratio.

| | |
|---|---|
| 500 mM K₃Fe(CN)₆ shading) | 50 µL (stored at room temperature, under |
| 500 mM K₄Fe(CN)₆ shading) | 50 µL (stored at room temperature, under |
| 1 M MgCl₂ | 10 µL (stored at room temperature) |
| 20 mg/mL X-gal | 250 µL (stored at -20°C, under shading) |
| PBS | 4.7 mL (stored at room temperature) |

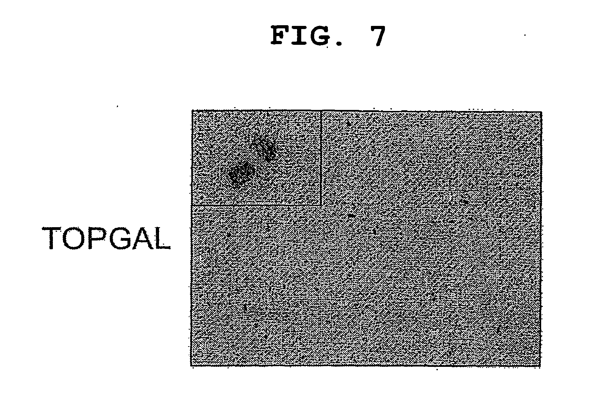

X-gal-positive cells were detected, confirming β catenin transcription activation (FIG. 7).

### Example 7: Induction of differentiation into cells that configure intestinal epithelial tissue

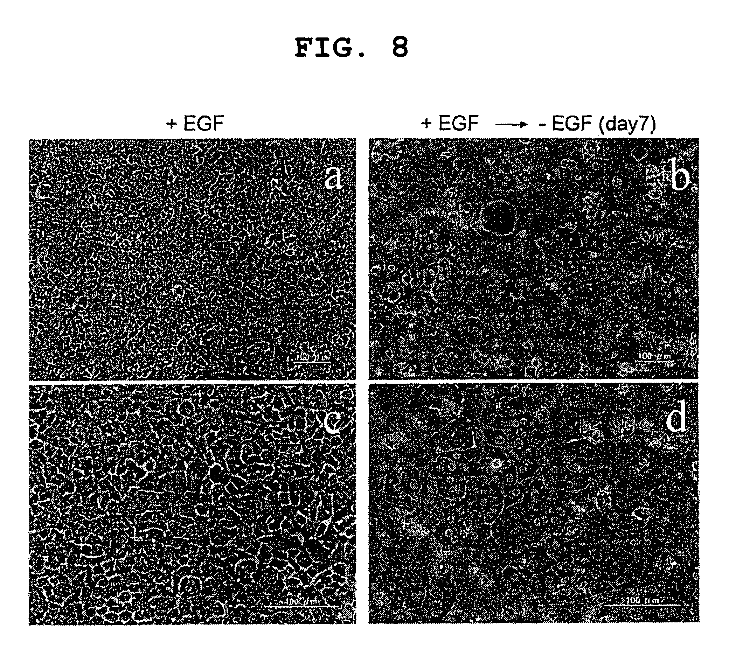

FIP cells were cultured until they became confluent. When EGF was removed from the confluent state, most of the cells underwent apoptosis. There were some survived. When these surviving cells were continued to be cultured in the absence of EGF, cells having characteristics of differentiated intestinal epithelium-like cells emerged (FIG. 8).

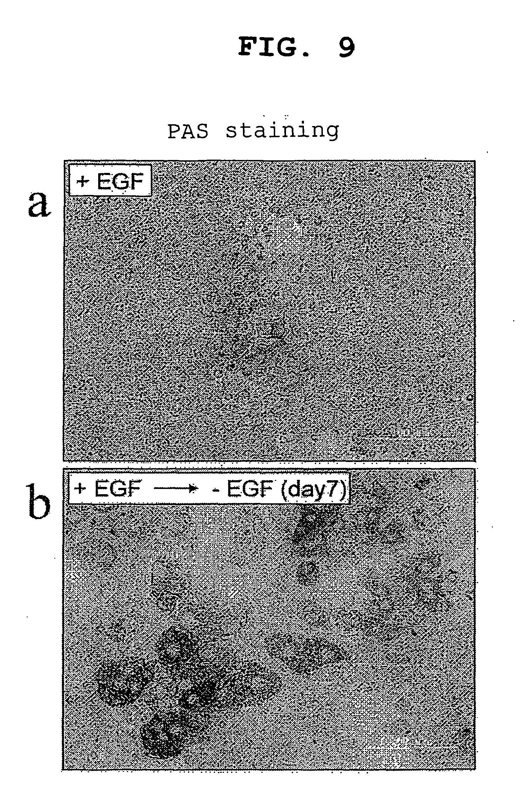

When the cells in this state were stained with PAS, a large number of red-purple-stained cells emerged out of the cells continued to be cultured in the absence of EGF (FIG. 9). PAS staining is based on two reactions to form a red-purple compound, i.e., oxidation of the group of 1,2-glycol groups contained in glucides with periodic acid, and binding of the resulting two molecules of aldehyde groups with one molecule of Schiff's reagent, and is used to detect glycogen, neutral mucopolysaccharides, glycoproteins, mucoproteins, glycolipids and the like. PAS staining is known to selectively stain goblet cells, a kind of mucus-secreting cell, in intestinal epithelial tissue. Specifically, staining was performed per the procedures shown below. Reagents in common use for PAS staining in the art can be used.
(1) Wash cells with PBS (X1).
(2) Treat with 0.5% periodic acid at room temperature for 7 minutes.
(3) Wash with Milli-Q water (X5).
(4) Treat with Schiff's reagent at room temperature for 7 to 10 minutes.
(5) Treat with aqueous sulfurous acid (repeat treatment at room temperature for 1 minute three times).
(6) Wash with Milli-Q water (X5).
(7) Treat with hematoxylene (at room temperature for 1 minute).
(8) Wash with Milli-Q water (X5).

Immunological staining was performed using antibodies against the proteins secreted and expressed in respective intestinal epithelial tissues, and the expression profiles of the proteins were analyzed. The antibodies used for the respective proteins are shown below. In order to visualize the presence of the cells, the cells were double-stained with the nucleus staining agent DAPI.
(1st antibodies)
   Anti-serotonin antibody: Immunostar, 20080 (2nd antibody, for rabbits); X10
   Anti-chromogranin A antibody: Santa Cruz, SC-1488 (2nd antibody, for goat); X10
   Anti-gastrin antibody: Santa Cruz, SC-7783 (2nd antibody, for goat); X10
   Anti-secretin antibody: Chemicon, AB981 (2nd antibody, for rabbits); X100
   Anti-somatostatin antibody: AFFINITI, SA1268 (2nd antibody, for rabbits); X10
   Anti-GIP antibody: Chemicon, AB953 (2nd antibody, for rabbits); X10
   Anti-lysozyme antibody: Dako, A0099 (2nd antibody, for rabbits); X100
   Anti-synaptophysin antibody: Dako, A0010 (2nd antibody, for rabbits); X10
(2nd antibodies)
   For rabbits: Alexa 488-conjugated donkey anti-rabbit IgG (Molecular Probes, A21206); X200
   For goat: Alexa 488-conjugated donkey anti-goat IgG (Molecular Probes, A11055); X200

As a result, when the cells were cultured in the absence of EGF, cells that were expressing serotonin, chromogranin A, gastrin, secretin, somatostatin, and GIP, which are characteristic of intestinal secretory cells, were detected, and cells that were expressing lysozyme and synaptophysin, which are characteristic of Paneth's cells, a kind of mucus-secreting cell, were detected. From these results, it is seen that by removing EGF from the FIP cell culture broth, differentiation into intestinal secretory cells was induced.

### Example 8: Analysis by RT-PCR

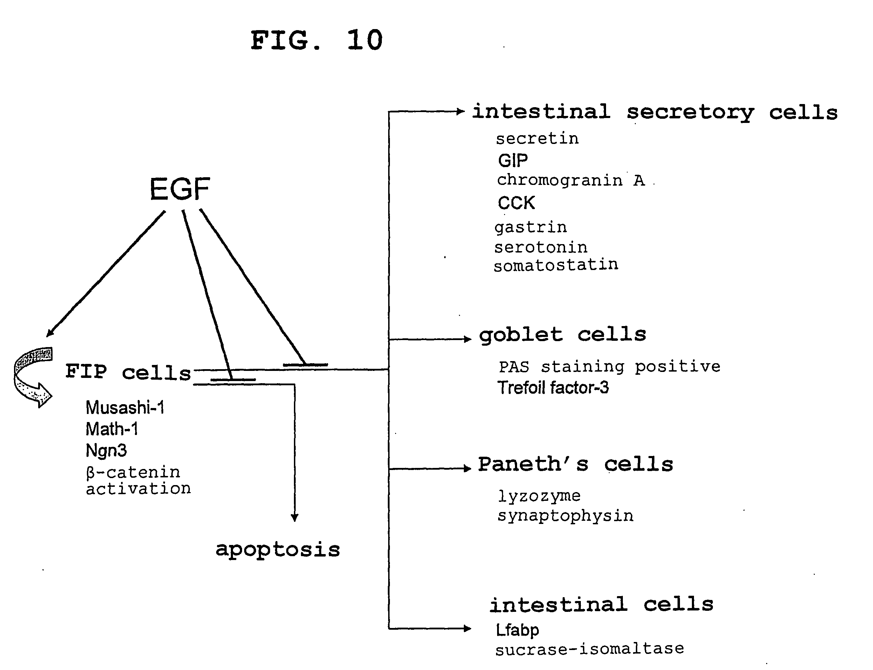

From the Examples shown above, it was shown that FIP cells included intestinal stem/progenitor cells, and it was found that in the presence of EGF, the stem/progenitor cells continued to proliferate, and that by removing EGF from the culture broth, the cells underwent apoptosis or differentiated into cells that configure various intestinal epithelial tissues (a schematic diagram shown in FIG. 10).

Using FIP cells and intestine tissues from mice at various developmental stages (day 14.5 of viviparity, neonates, adults), the expression profiles of genes thought to be involved in intestinal cell differentiation were analyzed by RT-PCR.

After FIP cells were cultured in EGF(+) medium until they became confluent, the medium was exchanged with an EGF-free medium, and the cells were thereafter cultured for 1 week. RNA was extracted from the cells (RNeasy Mini Kit; QIAGEN), and cDNAs were obtained by a reverse transcriptase reaction (SuperScript III). In RNA extraction from intestine tissues, each tissue was disrupted using a homogenizer (Polytron), after which extraction and a reverse transcriptase reaction were performed in the same manner. Subsequently, PCR was performed using the cDNAs thereof, and the expression of various differentiation markers was analyzed.

Desired markers and primers therefor are shown in Table 1.

**Table 1**

| Marker | Primer | |
|---|---|---|
| | Forward | Reverse |
| Villin | SEQ ID NO:1 | SEQ ID NO:2 |
| Lfabp | SEQ ID NO:3 | SEQ ID NO:4 |
| Secretin | see Suzuki A., et al., J Cell Biol, 156, 173-184, 2002 | |
| GIP | see Jensen, J. et al., Nat. Genet. 24: 36-44, 2000 | |
| Gastrin | see Jensen, J. et al., Nat. Genet. 24: 36-44, 2000 | |
| Chromogranin A | SEQ ID NO:5 | SEQ ID NO:6 |
| CCK | see Jensen, J. et al., Nat. Genet. 24: 36-44, 2000 | |
| Somatostatin | see Suzuki A., et al., J Cell Biol, 156, 173-184, 2002 | |
| Trefoil factor-3 | SEQ ID NO: 7 | SEQ ID NO: 8 |
| Lysozyme | SEQ ID NO: 9 | SEQ ID NO: 10 |
| Synaptophysin | SEQ ID NO: 11 | SEQ ID NO: 12 |
| Musashi-1 | SEQ ID NO: 13 | SEQ ID NO: 14 |
| Math-1 | SEQ ID NO: 15 | SEQ ID NO: 16 |
| Ngn3 | see Suzuki A., et al., PNAS USA, 100, 5034-5039, 2003 | |
| HPRT (internal standard) | see Suzuki A., et al., Hepatology, 32, 1230-1239, 2000 | |

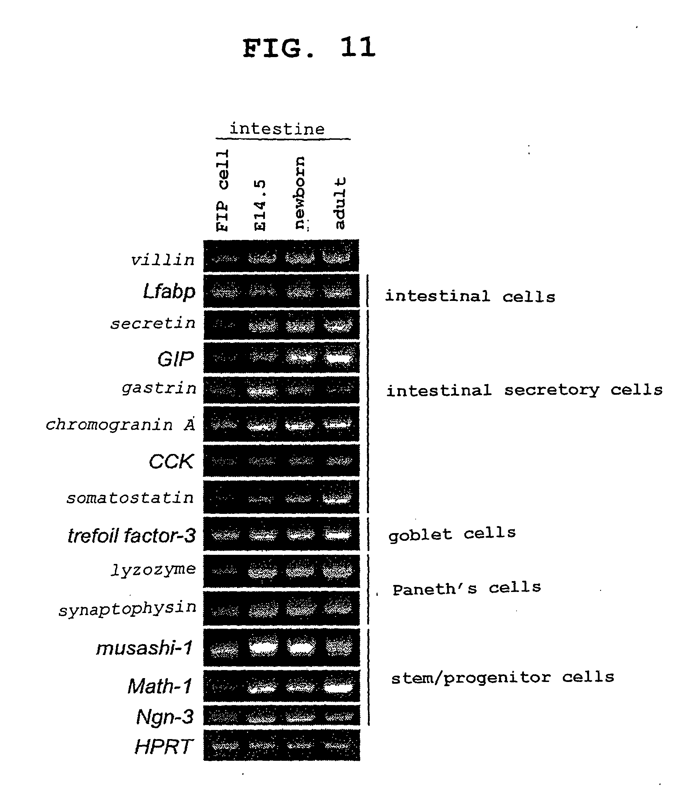

The PCR cycle comprised 95°C (4 minutes)→[94°C (1 minute)→56°C (1 minute)→72°C (1 minute)] X45 cycles →72°C (10 minutes). The nucleic acids produced by the PCR were developed on 2% agarose gel, and analyzed. The results are shown in FIG. 11.

### Example 9: Analysis at the single-cell level

Since the above-described experiments in Examples 3 to 7 comprised analyses of a heterogeneous population of cells, we thought that analyses at the single-cell level were necessary to accurately analyze intestinal stem/progenitor cells. Hence, FIP cells were subjected to clone sorting using flowcytometry, and single-cell culture was attempted in each well of a 96-well dish. Using one of the clones that had proliferated, proliferation capability, the expression of marker proteins and differentiation induction were examined in the same manner.

As a result, even in the cells proliferated from a single colony, E-cadherin, Zo-1, and villin were expressed in all cells, with some colonies expressing Musashi-1. Furthermore, the cells exhibited EGF-dependent proliferation activity and cell death suppression, and came to express a plurality of intestinal epithelial cell markers in the absence of EGF. Hence, proliferation capability and pluripotency were demonstrated even in the analyses at the single-cell level, confirming that the FIP cells contained intestinal stem/progenitor cells.

From these results, it was shown that the population of FIP cells comprised stem/progenitor cells having self-replicating capability and pluripotency, and could be stably maintained for a long time. By using the method of the invention of this application, it has become possible to proliferate stem/progenitor cells of intestinal epithelial cells selectively. By this selective proliferation, intestinal stem/progenitor cells can efficiently be acquired and cultured.

### Example 10: Acquirement of intestinal stem/progenitor cells from human small intestine epithelial cells

Used in this Example were human small intestine epithelial cells (Cat No. CS-ABI-519) separated by ACBRI, and supplied in Japan by Dainippon Sumitomo Pharma Co., Ltd. These cells were sown to a culture dish and cultured according to the method described in the attached data sheet (FIG. 12a). The culture dish used was a dish coated with type IV collagen. Used as the culture broth was the attached medium described in the data sheet, i.e., CS-2.0 basal medium supplemented with additives (hereinafter, CS-2.0 + supplement). This treatment was performed in the presence of 20 ng/mL EGF.

When cultured using CS-2.0 + supplement, all cells died, without adhering to the culture dish, at the time of completion of 2 times of passage (FIGS. 12c and e).

Hence, the cells were subcultured in the same manner, but using the medium containing EGF for culturing fetal mouse intestinal cells (medium for intestinal stem/progenitor cell culture), prepared in Example 1; cells capable of adhering to the culture dish and maintaining proliferation even subjected to many passages were obtained (FIGS. 12b and d).

Since these cells were thought to be intestinal stem/progenitor cells contained in the purchased population of human epithelial cells, they are hereinafter also referred to as hAIP (human adult intestine progenitor) cells for the sake of convenience.

### Example 11: Marker protein expression and β catenin transcription activation

hAIP cells were examined for the expression of marker proteins in the same manner as Example 5.

All hAIP cells expressed E-cadherin and Zo-1, markers of epithelial cells, and villin, a marker of intestinal epithelial cells. Some cells expressed Musashi-1, which is thought to be an intestinal stem/progenitor cell marker (FIG. 13, upper panel).

hAIP cells were also examined for the presence or absence of β catenin transcription activation in the same manner as Example 6. X-gal-positive cells were detected, confirming β catenin transcription activation (FIG. 13, lower panel).

### Example 12: Proliferation capability

The proliferation capability of hAIP cells was examined in the same manner as Example 3. The cells were cultured in the EGF(+) medium used in Example 1, and in an EGF(-) medium of the same composition, but not containing EGF, for 4 days. hAIP cells exhibited vigorous division and proliferation in the presence of EGF; however, under EGF-free culture conditions, the proliferation activity decreased, and cells undergoing apoptosis increased (FIG. 14).

From this result, it is seen that EGF promotes the self-replication of hAIP cells and suppresses cell death to thereby enable the maintenance thereof in culture.

### Example 13: Proliferation and apoptosis

hAIP cells were cultured in EGF(+) medium (EGF concentration 20 ng/mL) for 4 days. Two sets in the same state were provided. After the cells were cultured in the presence of EGF for 4 days, for one set, the medium was exchanged with the same EGF(+) medium and the cells were further cultured for 1 day (FIG. 15A, left); for the other set, the medium was exchanged with an EGF-free culture broth and the cells were further cultured for 1 day (FIG. 15A, right). The cells continued to proliferate in the presence of EGF; however, in the absence of EGF, the proliferation of the cells was suppressed, and dead cells were observed. Changes in the proliferation capability of the cells in the series of processes were confirmed by the BrdU immunological staining method. The cells were double-stained with the nucleus staining agent DAPI (FIG. 15A).

After the cells were cultured in the presence of EGF for 4 days, for one set, the medium was exchanged with an EGF-free culture broth and the cells were further cultured for 1 day; for the other set, the medium was exchanged with the same EGF(+) medium and the cells were further cultured for 1 day. Thereafter, BrdU (10 µM) (Sigma, B9285) was added to the medium; 1.5 hours later, the ratio of proliferated cells that had incorporated BrdU was analyzed by flowcytometry and immunological staining. Flowcytometry analysis was performed according to the method described in Example 4 (FIG. 15B, left). The cells were cultured as with the BrdU analysis, and binding with annexin V was analyzed according to the method described in Example 4. The ratio of cells to which annexin V binds, deemed cells undergoing apoptosis, was examined (FIG. 15B, right). It is seen that by removing EGF from the culture broth, BrdU uptake decreases and hence the proliferation capability decreases. Furthermore, by removing EGF from the culture broth, cells undergoing apoptosis increased.

To determine the effects of EGF on the proliferation or apoptosis of hAIP cells, BrdU uptake and apoptosis induction were examined in the same manner using a culture broth prepared by adding AG1478, PD98059, or LY294002, which inhibit the EGF signal transduction system, to a culture broth containing EGF.

PD98059 (2'-amino-3'-methoxyflavon), a selective cell-permeable inhibitor of MEK1 (MAP kinase kinase), inhibits MEK1 activation and, as a result, inhibits the subsequently occurring phosphorylation-activation of MAPK (ERK1/2). Concentration used: 100 µM.

LY294002 ([2-(4-morpholinyl)-8-phenyl-4H-1-benzopyran-4-one] is a potent specific cell permeable inhibitor of phosphatidylinositol 3-kinase (PI3-kinase). Concentration used: 100 µM

### Example 14: Induction of differentiation into cells that configure intestinal epithelial tissue

hAIP cells undergo spontaneous induction of differentiation under the above-described culture conditions, resulting in the emergence of cells showing characteristics of intestinal epithelium-like cells. When the cells in this state were subjected to PAS staining according to the method described in Example 7, a large number of red-purple stained cells emerged, confirming the presence of goblet cells, which is an intestinal epithelial tissue (FIG. 16B).

Furthermore, by the same technique as Example 8, induction of differentiation into cells that configure intestinal epithelial tissue was examined by RT-PCR analysis. The kinds of intestinal epithelial tissues induced are shown in FIG. 10.

Using hAIP cells and human adult small intestine tissue, the expression profiles of genes thought to be involved in intestinal cell differentiation were analyzed by RT-PCR. Desired markers and primers therefor used are those for human use shown in Table 2 below.

**Table 2**

| Marker | Primer | |
|---|---|---|
| | Forward | Reverse |
| Villin | SEQ ID NO: 17 | SEQ ID NO: 18 |
| Lfabp | SEQ ID NO: 19 | SEQ ID NO: 20 |
| Sucrase-isomaltase | SEQ ID NO: 21 | SEQ ID NO: 22 |
| Secretin | SEQ ID NO: 23 | SEQ ID NO: 24 |
| GIP | SEQ ID NO: 25 | SEQ ID NO: 26 |
| Gastrin | SEQ ID NO: 27 | SEQ ID NO: 28 |
| Chromogranin A | SEQ ID NO: 29 | SEQ ID NO: 30 |
| CCK | SEQ ID NO: 31 | SEQ ID NO: 32 |
| Somatostatin | SEQ ID NO: 33 | SEQ ID NO: 34 |
| Trefoil factor-3 | SEQ ID NO: 35 | SEQ ID NO: 36 |
| Lysozyme | SEQ ID NO: 37 | SEQ ID NO: 38 |
| Synaptophysin | SEQ ID NO: 39 | SEQ ID NO: 40 |
| Musashi-1 | SEQ ID NO: 41 | SEQ ID NO: 42 |
| Math-1 | SEQ ID NO: 43 | SEQ ID NO: 44 |
| Ngn3 | SEQ ID NO: 45 | SEQ ID NO: 46 |
| HPRT (internal standard) | SEQ ID NO: 47 | SEQ ID NO: 48 |

The results are shown in FIG. 16A.

Observed during hAIP cell culture were a plurality of types of cells that configure intestinal epithelial tissue, showing characteristics of intestinal secretory cells and mucus-secreting cells. Therefore, hAIP cells were found to have pluripotency for differentiating into a variety of cells that configure the intestine while continuing to proliferate in culture.

### Sequence Listing Free Text

Sequence No. 1: PCR primer for detection of Villin (mouse) (forward)
Sequence No. 2: PCR primer for detection of Villin (mouse) (reverse)
Sequence No. 3: PCR primer for detection of Lfabp (mouse) (forward)
Sequence No. 4: PCR primer for detection of Lfabp (mouse) (reverse)
Sequence No. 5: PCR primer for detection of Chromogranin A (mouse) (forward)
Sequence No. 6: PCR primer for detection of Chromogranin A (mouse) (reverse)
Sequence No. 7: PCR primer for detection of Trefoil factor-3 (mouse) (forward)
Sequence No. 8: PCR primer for detection of Trefoil factor-3 (mouse) (reverse)
Sequence No. 9: PCR primer for detection of Lysozyme (mouse) (forward)
Sequence No. 10: PCR primer for detection of Lysozyme (mouse) (reverse)
Sequence No. 11: PCR primer for detection of Synaptophysin (mouse) (forward)
Sequence No. 12: PCR primer for detection of Synaptophysin (mouse) (reverse)
Sequence No. 13: PCR primer for detection of Musashi-1 (mouse) (forward)
Sequence No. 14: PCR primer for detection of Musashi-1 (mouse) (reverse)
Sequence No. 15: PCR primer for detection of Math-1 (mouse) (forward)
Sequence No. 16: PCR primer for detection of Math-1 (mouse) (reverse)
Sequence No. 17: PCR primer for detection of Villin (human) (forward)
Sequence No. 18: PCR primer for detection of Villin (human) (reverse)
Sequence No. 19: PCR primer for detection of Lfabp (human) (forward)
Sequence No. 20: PCR primer for detection of Lfabp (human) (reverse)
Sequence No. 21: PCR primer for detection of Sucrase-isomaltase (human) (forward)
Sequence No. 22: PCR primer for detection of Sucrase-isomaltase (human) (reverse)
Sequence No. 23: PCR primer for detection of Secretin (human) (forward)
Sequence No. 24: PCR primer for detection of Secretin (human) (reverse)
Sequence No. 25: PCR primer for detection of GIP (human) (forward)
Sequence No. 26: PCR primer for detection of GIP (human) (reverse)
Sequence No. 27: PCR primer for detection of Gastrin (human) (forward)
Sequence No. 28: PCR primer for detection of Gastrin (human) (reverse)
Sequence No. 29: PCR primer for detection of Chromogranin A (human) (forward)
Sequence No. 30: PCR primer for detection of Chromogranin A (human) (reverse)
Sequence No. 31: PCR primer for detection of CCK (human) (forward)
Sequence No. 32: PCR primer for detection of CCK (human) (reverse)
Sequence No. 33: PCR primer for detection of Somatostatin (human) (forward)
Sequence No. 34: PCR primer for detection of Somatostatin (human) (reverse)
Sequence No. 35: PCR primer for detection of Trefoil factor-3 (human) (forward)
Sequence No. 36: PCR primer for detection of Trefoil factor-3 (human) (reverse)
Sequence No. 37: PCR primer for detection of Lysozyme (human) (forward)
Sequence No. 38: PCR primer for detection of Lysozyme (human) (reverse)
Sequence No. 39: PCR primer for detection of Synaptophysin (human) (forward)
Sequence No. 40: PCR primer for detection of Synaptophysin (human) (reverse)
Sequence No. 41: PCR primer for detection of Musashi-1 (human) (forward)
Sequence No. 42: PCR primer for detection of Musashi-1 (human) (reverse)
Sequence No. 43: PCR primer for detection of Math-1 (human) (forward)
Sequence No. 44: PCR primer for detection of Math-1 (human) (reverse)
Sequence No. 45: PCR primer for detection of Ngn3 (Neurogenin-3) (human) (forward)
Sequence No. 46: PCR primer for detection of Ngn3 (Neurogenin-3) (human) (reverse)
Sequence No. 47: PCR primer for detection of HPRT (human) (forward)
Sequence No. 48: PCR primer for detection of HPRT (human) (reverse)

### Industrial Applicability

Stem/progenitor cells of intestinal epithelial cells obtained by the present invention and a method of inducing differentiation from the cells to cells that configure intestinal epithelial tissue are useful as basic research tools for understanding the genesis and differentiation of intestine tissue, and make it possible to supply intestinal epithelial tissue that can be transplantable by tissue engineering. Furthermore, it is expected that using intestinal epithelial cells derived from the stem/progenitor cells, experiments of drug absorption in the intestine and the like can efficiently be performed.

This application is based on a patent application Nos. 2007-024169 (filing date: February 2, 2007) and 2007-241536 (filing date: September 18, 2007) filed in Japan, the contents of which are incorporated in full herein by this reference.

## Claims

1. A method of acquiring stem/progenitor cells of intestinal epithelial cells, comprising culturing an aggregate of intestinal cells.

2. A method of acquiring stem/progenitor cells of intestinal epithelial cells, comprising culturing an aggregate of intestinal cells in a culture broth containing EGF.

3. The method according to claim 1 or 2, wherein the aggregate is obtained by suspension-culturing intestinal cells.

4. A method of acquiring stem/progenitor cells of intestinal epithelial cells, comprising the following steps:
(1) a step for collecting intestinal cells from the intestine;
(2) a step for preparing a cell mass (aggregate) by suspension-culturing intestinal cells;
(3) a step for culturing an aggregate of intestinal cells in a culture broth containing EGF; and
(4) a step for recovering cells that are expressing a marker for stem/progenitor cell of intestinal epithelial cells as stem/progenitor cells of intestinal epithelial cells.

5. The method according to claim 4, wherein the marker for stem/progenitor cells of intestinal epithelial cells is Musashi-1.

6. A method of acquiring stem/progenitor cells of intestinal epithelial cells, comprising culturing intestinal cells in a culture broth containing serum, insulin, dexamethasone, nicotinamide, L-glutamine, β-mercaptoethanol and EGF.

7. A method of acquiring stem/progenitor cells of intestinal epithelial cells, comprising culturing intestinal cells at high density in a culture broth containing EGF.

8. A method of acquiring stem/progenitor cells of intestinal epithelial cells, comprising the following steps:
(1) a step for collecting intestinal cells from the intestine;
(2) a step for culturing intestinal cells at high density in a culture broth containing EGF; and
(3) A step for recovering cells that are expressing a marker for stem/progenitor cell of intestinal epithelial cells as stem/progenitor cells of intestinal epithelial cells.

9. The method according to claim 8, wherein the marker for stem/progenitor cell of intestinal epithelial cells is Musashi-1.

10. Stem/progenitor cells of intestinal epithelial cells obtained by the method according to any one of claims 1 to 9.

11. A method of culturing stem/progenitor cells of intestinal epithelial cells, comprising culturing an aggregate of intestinal cells.

12. A method of culturing stem/progenitor cells of intestinal epithelial cells, comprising culturing an aggregate of intestinal cells in a culture broth containing EGF.

13. The method according to claim 11 or 12, wherein the aggregate is obtained by suspension-culturing intestinal cells.

14. A method of culturing stem/progenitor cells of intestinal epithelial cells, comprising culturing intestinal cells in a culture broth containing serum, insulin, dexamethasone, nicotinamide, L-glutamine, β-mercaptoethanol and EGF.

15. A method of culturing stem/progenitor cells of intestinal epithelial cells, comprising culturing intestinal cells at high density in a culture broth containing EGF.

16. A method of inducing differentiation from stem/progenitor cells of intestinal epithelial cells to cells that configure intestinal epithelial tissue, wherein EGF is removed from a culture broth of the stem/progenitor cells.

17. The method according to claim 16, wherein the cells that configure intestinal epithelial tissue are intestinal secretory cells or mucus-secreting cells.

18. A medium for culturing stem/progenitor cells of intestinal epithelial cells, containing serum, insulin, dexamethasone, nicotinamide, L-glutamine, β-mercaptoethanol and EGF.


SEQUENCE LISTING

<110> RIKEN

<120> Method for Obtaining Intestinal Stem/Precursor Cell

<130> 1808 N.103982

<150> JP2007-024169
<151> 2007-02-02

<150> JP2007-241536
<151> 2007-09-18

<160> 48

<170> PatentIn version 3.3

<210> 1
<211> 21
<212> DNA
<213> Artificial

<220>
<223> PCR primer for detection of Villin(mouse) (foward)

<400> 1
ttcgatggtg actgctatgt a 21

<210> 2
<211> 21
<212> DNA
<213> Artificial

<220>
<223> PCR primer for detection of Villin(mouse) (reverse)

<400> 2
tcagaaatag cagccttcag t 21

<210> 3
<211> 19
<212> DNA
<213> Artificial

<220>
<223> PCR primer for detection of Lfabp(mouse) (forward)

<400> 3
agaactttga gccattcat 19

<210> 4
<211> 19
<212> DNA
<213> Artificial

<220>
<223> PCR primer for detection of Lfabp(mouse) (reverse)

<400> 4
tgactctctt gtagacaat 19

<210> 5
<211> 21
<212> DNA
<213> Artificial

<220>
<223> PCR primer for detection of Chromogranin A (mouse)(forward)

<400> 5
tctatcctgc gacaccagaa t 21

<210> 6
<211> 21
<212> DNA
<213> Artificial

<220>
<223> PCR primer for detection of Chromogranin A(mouse) (reverse)

<400> 6
tggaagtggg aactggatgc t 21

<210> 7
<211> 22
<212> DNA
<213> Artificial

<220>
<223> PCR primer for detection of Trefoil factor-3 (mouse)(forward)

<400> 7
agaccagagc cctctggcta at 22

<210> 8
<211> 22
<212> DNA
<213> Artificial

<220>
<223> PCR primer for detection of Trefoil factor-3(mouse) (reverse)

<400> 8
tgtgcattct gtctcctgca ga 22

<210> 9
<211> 20
<212> DNA
<213> Artificial

<220>
<223> PCR primer for detection of Lysozyme (mouse)(forward)

<400> 9
aaggtctatg aacgttgtga 20

<210> 10
<211> 20
<212> DNA
<213> Artificial

<220>
<223> PCR primer for detection of Lysozyme(mouse) (reverse)

<400> 10
gaatatactg ggacagatct 20

<210> 11
<211> 20
<212> DNA
<213> Artificial

<220>
<223> PCR primer for detection of Synaptophysin (mouse)(foward)

<400> 11
atcgaagtcg aatttgagta 20

<210> 12
<211> 20

<212>  DNA
<213>  Artificial

<220>
<223>  PCR primer for detection of Synaptophysin (mouse)(reverse)

<400>  12
ttgaacacga accataggtt                                                    20


<210>  13
<211>  21
<212>  DNA
<213>  Artificial

<220>
<223>  PCR primer for detection of Musashi-1 (mouse)(foward)

<400>  13
tgatgccatg ctgatgttcg a                                                  21


<210>  14
<211>  19
<212>  DNA
<213>  Artificial

<220>
<223>  PCR primer for detection of Musashi-1(mouse) (reverse)

<400>  14
tgaaggctgt ggcaatcaa                                                     19


<210>  15
<211>  20
<212>  DNA
<213>  Artificial

<220>
<223>  PCR primer for detection of Math-1(mouse) (forward)

<400>  15
agaagagtgg gctgaggtaa                                                    20


<210>  16
<211>  20
<212>  DNA
<213>  Artificial

<220>
<223>  PCR primer for detection of Math-1(mouse) (reverse)

<400>  16
ttgaaggacg ggataacgtt                                                    20


<210>  17
<211>  20
<212>  DNA
<213>  Artificial

<220>
<223>  PCR primer for detection of Villin (human) (forward)

<400>  17
ttcgatggtg actgctacat                                                    20


<210>  18
<211>  20
<212>  DNA
<213>  Artificial

<220>

<223>  PCR primer for detection of Villin (human)(reverse)

<400>  18
ttcatcacct ccatcagctt                                                    20


<210>  19
<211>  21
<212>  DNA
<213>  Artificial

<220>
<223>  PCR primer for detection of Lfabp (human)(foward)

<400>  19
cttcatgaag gcaatcggtc t                                                  21


<210>  20
<211>  21
<212>  DNA
<213>  Artificial

<220>
<223>  PCR primer for detection of Lfabp(human)(reverse)

<400>  20
gaagacaatg tcacccaatg t                                                  21


<210>  21
<211>  22
<212>  DNA
<213>  Artificial

<220>
<223>  PCR primer for detection of Sucrase-isomaltase(human) (forward)

<400>  21
actctcttat tccttggtgc tt                                                 22


<210>  22
<211>  22
<212>  DNA
<213>  Artificial

<220>
<223>  PCR primer for detection of Sucrase-isomaltase (human)(reverse)

<400>  22
attatcacca ggaagttggt ct                                                 22


<210>  23
<211>  20
<212>  DNA
<213>  Artificial

<220>
<223>  PCR primer for detection of Secretin (human)(forward)

<400>  23
acactcagac gggacgttca                                                    20


<210>  24
<211>  20
<212>  DNA
<213>  Artificial

<220>
<223>  PCR primer for detection of Secretin(human) (reverse)

<400>  24
atctgggccg caaggttcct                                                    20

<210>  25
<211>  20
<212>  DNA
<213>  Artificial

<220>
<223>  PCR primer for detection of GIP(human) (forward)

<400>  25
tgttcctggc agtgggacta                                                    20


<210>  26
<211>  21
<212>  DNA
<213>  Artificial

<220>
<223>  PCR primer for detection of GIP (human)(reverse)

<400>  26
tgagcctgca gaggtttgtc t                                                  21


<210>  27
<211>  20
<212>  DNA
<213>  Artificial

<220>
<223>  PCR primer for detection of Gastrin (human)(forward)

<400>  27
ctgatctttg cactggctct                                                    20


<210>  28
<211>  20
<212>  DNA
<213>  Artificial

<220>
<223>  PCR primer for detection of Gastrin(human) (reverse)

<400>  28
agttctcatc ctcagcactg                                                    20


<210>  29
<211>  20
<212>  DNA
<213>  Artificial

<220>
<223>  PCR primer for detection of Chromogranin A(human) (forward)

<400>  29
gaggagatga acggatcctt                                                    20


<210>  30
<211>  20
<212>  DNA
<213>  Artificial

<220>
<223>  PCR primer for detection of Chromogranin A(human) (reverse)

<400>  30
cctttccgga tctccttgta                                                    20


<210>  31
<211>  20

<212>  DNA
<213>  Artificial

<220>
<223>  PCR primer for detection of CCK(human) (forward)

<400>  31
tgcgtgctga tggcggtact                                                    20


<210>  32
<211>  20
<212>  DNA
<213>  Artificial

<220>
<223>  PCR primer for detection of CCK(human) (reverse)

<400>  32
tactcatact cctcggcact                                                    20


<210>  33
<211>  21
<212>  DNA
<213>  Artificial

<220>
<223>  PCR primer for detection of Somatostatin(human) (forward)

<400>  33
actccgtcag tttctgcaga a                                                  21


<210>  34
<211>  21
<212>  DNA
<213>  Artificial

<220>
<223>  PCR primer for detection of Somatostatin (human)(reverse)

<400>  34
acaggatgtg aaagtcttcc a                                                  21


<210>  35
<211>  20
<212>  DNA
<213>  Artificial

<220>
<223>  PCR primer for detection of Trefoil factor-3 (human)(forward)

<400>  35
tccagctctg ctgaggagta                                                    20


<210>  36
<211>  20
<212>  DNA
<213>  Artificial

<220>
<223>  PCR primer for detection of Trefoil factor-3 (human)(reverse)

<400>  36
gaaggtgcat tctgcttcct                                                    20


<210>  37
<211>  21
<212>  DNA
<213>  Artificial

<220>

<223>  PCR primer for detection of Lysozyme (human)(forward)

<400>  37
tgtctgctca gctgcctgct a                                                21


<210>  38
<211>  21
<212>  DNA
<213>  Artificial

<220>
<223>  PCR primer for detection of Lysozyme (human)(reverse)

<400>  38
aagtcacagc catccaccca t                                                21


<210>  39
<211>  21
<212>  DNA
<213>  Artificial

<220>
<223>  PCR primer for detection of Synaptophysin (human)(forward)

<400>  39
tgcaatgggt cttcgccatc t                                                21


<210>  40
<211>  21
<212>  DNA
<213>  Artificial

<220>
<223>  PCR primer for detection of Synaptophysin(human) (reverse)

<400>  40
cacaaggtct ctcagctcct t                                                21


<210>  41
<211>  20
<212>  DNA
<213>  Artificial

<220>
<223>  PCR primer for detection of Musashi-1(human) (forward)

<400>  41
ttcggcttcg tcactttcat                                                  20


<210>  42
<211>  21
<212>  DNA
<213>  Artificial

<220>
<223>  PCR primer for detection of Musashi-1(human) (reverse)

<400>  42
tgtataactc cggctggcgt a                                                21


<210>  43
<211>  19
<212>  DNA
<213>  Artificial

<220>
<223>  PCR primer for detection of Math-1(human) (forward)

<400>  43
gcaatgttat cccgtcgtt                                                   19

<210> 44
<211> 19
<212> DNA
<213> Artificial

<220>
<223> PCR primer for detection of Math-1(human) (reverse)

<400> 44
gaaagtcgag aagtgcaga 19


<210> 45
<211> 20
<212> DNA
<213> Artificial

<220>
<223> PCR primer for detection of Ngn3(Neurogenin-3)(human) (forward)

<400> 45
taagagcgag ttggcactga 20


<210> 46
<211> 21
<212> DNA
<213> Artificial

<220>
<223> PCR primer for detection of Ngn3(Neurogenin-3) (human)(reverse)

<400> 46
tctgagaaag ccagactgcc t 21


<210> 47
<211> 20
<212> DNA
<213> Artificial

<220>
<223> PCR primer for detection of HPRT(human) (forward)

<400> 47
acaggactga acgtcttgct 20


<210> 48
<211> 20
<212> DNA
<213> Artificial

<220>
<223> PCR primer for detection of HPRT (human)(reverse)

<400> 48
gtctggctta tatccaacac 20